# FASCICULE DE BREVET EUROPEEN

(11) **EP 1 456 352 B1**
(45) Date de publication et mention de la délivrance du brevet: **27.04.2005**
(21) Numéro de dépôt: 02793186.4
(22) Date de dépôt: 24.10.2002
(51) Int. Cl.: C12N 5/00, C08B 37/00

(54) **MILIEU DE CULTURE SOLIDE POUR MICRO-ORGANISMES ET CELLULES EUCARYOTES AINSI QUE SON PROCEDE D'OBTENTION**
FESTES KULTURMEDIUM FÜR MIKROORGANISMEN UND EUKARYONTISCHE ZELLEN UND VERFAHREN ZU SEINER HERSTELLUNG
SOLID CULTURE MEDIUM FOR MICRO-ORGANISMS AND EUKARYOTIC CELLS AND METHOD FOR ITS PRODUCTION

(30) Priorité: 29.10.2001 FR 0113993; 21.10.2002 FR 0213095
(43) Date de publication de la demande: 15.09.2004
(73) Titulaire: AGRO INDUSTRIE RECHERCHES ET DEVELOPPEMENTS (A.R.D.), 51110 Pomacle (FR)
(72) Inventeur: BELARDI, Abdelkader, F-51430 Bezannes (FR); DE BAYNAST, Régis, F-78000 Versailles (FR); BRESIN, Anthony, F-51110 Reims (FR)
(74) Mandataire: Hammond, William
(86) Numéro de dépôt international: PCT/FR2002/003659
(87) Numéro de publication internationale: WO 2003/038069

(56) Documents cités:
- WO-A-98/35993

## Description

La présente invention concerne un milieu de culture solide pour micro-organismes et cellules eucaryotes ainsi que son procédé d'obtention.

De nos jours l'ensemble des milieux de culture solides contient une base gélifiante communément appelée Agar-Agar. En raison des propriétés intrinsèques des milieux gélosés, ceux-ci trouvent de nombreuses applications dans des domaines aussi divers que la recherche fondamentale en biologie moléculaire, le génie génétique, les méthodes d'identification de micro-organismes, ainsi que la conservation de ceux-ci pour le maintien des collections.

Cependant, l'obtention de colonies à identifier prennent plusieurs jours quand, bien souvent, comme dans le cas d'échantillons médicaux, le temps est compté.

En ce qui concerne les collections de micro-organismes, l'appauvrissement rapide du milieu entraîne la nécessité d'un repiquage systématique fréquent.

Aussi, un des buts de la présente invention est-il de fournir un nouveau milieu de culture solide, dans lequel la biodisponibilité des substrats est optimale.

Ce but, ainsi que d'autres qui apparaîtront par la suite, est obtenu par un milieu de culture solide, caractérisé par le fait qu'il comprend, comme base gélifiante, un polysaccharide ayant une unité de reproduction qui possède une chaîne latérale et est constitué par six sucres neutres, dont le glucose et le galactose, et un sucre acide, des substituants pyruvates et acétates étant présents, une solution dans l'eau ou une solution saline à une concentration en ce polysaccharide supérieure ou égale-à 2g/l formant un gel élastique et transparent.

De préférence, ce polysaccharide est synthétisé par un Rhizobium déposé sous le N°I-1809 auprès de la CNCM.

Avantageusement, le milieu de culture comprend de 2,2 à 6 % en poids de ce polysaccharide par rapport au poids total de ce milieu.

De préférence, ce milieu de culture solide comprend aussi 0,5 M de chlorure de sodium (NaCl)

Ainsi qu'il a été dit précédemment, la présente invention concerne aussi un procédé pour obtenir ce milieu de culture, selon lequel :
a) On mélange des milieux nutritionnels connus sous forme de poudre ;
b) On ajoute à ce mélange de 2,2 à 6 % en poids du polysaccharide ci-dessus sous forme de poudre ;
c) On met en solution le mélange ainsi obtenu par adjonction d'eau en quantité suffisante afin de satisfaire aux recommandations pour le milieu considéré ;
d) On stérilise la solution issue de l'étape c) ; et,
e) On coule dans des récipients ad hoc le produit stérilisé encore chaud.

Avantageusement, à l'issue de l'étape e), on laisse reposer pendant 24 h à température ambiante.

De préférence, on ajoute, si nécessaire, à la solution issue de l'étape c) 0,5 M de chlorure de sodium (NaCl).

L'exemple de réalisation qui suit et qui ne présente aucun caractère limitatif, permettra à l'homme de métier de mieux comprendre la présente invention et notamment ses nombreux avantages.

On réalise un milieu de culture selon la présente invention à partir de la composition d'un milieu connu tel que celui dénommé Luria Bertani qui est commercialisée par la société Difco sous la référence 0446-17-3, complémenté d'un mélange cceur/cervelle commercialisée par la société OXOID sous la référence CM 225, dont la base gélifiante est de l'Agar-Agar ; mais on remplace celle-ci par un polysaccharide du genre comprenant une unité de reproduction qui possède une chaîne latérale et est constitué par six sucres neutres, dont le glucose et le galactose, et un sucre acide, des substituants pyruvates et acétates étant présents ; une solution dans l'eau ou une solution saline en ce polysaccharide à une concentration supérieure ou égale à 2g/l forme un gel élastique et transparent. Un tel polysaccharide est d'ailleurs décrit. dans la demande de brevet français publiée sous le N°2 759 377.

Pour réaliser ce milieu, on mélange les milieux nutritionnels sous forme de poudre puis on ajoute le polysaccharide sous forme de poudre dans une proportion comprise entre 2,2 et 6 % en poids. Puis on solubilise les poudres par adjonction d'eau en quantité suffisante afin de satisfaire aux recommandations pour le milieu considéré.

On soumet l'ensemble à une stérilisation par exemple à 110°C pendant 10 mn ou 121°C pendant 15 mn, à l'autoclave. Les différentes poudres n'ont pas besoin d'être parfaitement solubilisées avant l'étape de stérilisation, le polysaccharide se dissolvant au cours de la stérilisation : on obtient ainsi un milieu de culture selon la présente invention dénommé par la suite « milieu invention ».

On a aussi préparé un milieu traditionnel contenant de l'Agar-Agar comme seule base gélifiante, qui constitue le milieu témoin, et un milieu, dit milieu mixte, comportant un mélange d'Agar-Agar et de polysaccharide comme base gélifiante. Dans le tableau 1 ci-dessous on a regroupé les compositions de ces trois milieux de culture.

**Tableau 1**

| Composition | Milieu Témoin Concentration en g/l | Milieu invention Concentration en g/l | Milieu mixte Concentration en g/l |
|---|---|---|---|
| Tryptones | 10 | 10 | 10 |
| NaCl | 10 | 10 | 10 |
| Extrait de levure | 5 | 5 | 5 |
| Cceur de cervelle | 5 | 5 | 5 |
| Glucose | 2 | 2 | 2 |
| Agar-Agar | 22 | 0 | 1 |
| Polysaccharide | 0 | 22 | 22 |

Les milieux ci-dessus sont coulés après stérilisation dans des boîtes de pétri rondes d'une surface totale de 5 800 mm². Après séchage et une journée entière de maturation, on ensemence chacun de ces milieux par le dépôt de 2 µl d'un inoculum de chacun des micro-organismes suivants : *Pseudomonas aeruginosa, Bacilis subtilis*, et *Staphylococcus epidermidis,* ceux-ci étant en phase exponentielle de croissance.

Après acquisition numérique des boîtes de pétri sur lesquelles se développent les colonies, les photos sont imprimées puis les colonies sont découpées. Sachant que le papier est d'une densité de 80g/m², la mesure du poids que représentent les disques permet d'estimer la surface de la colonie. Ainsi, malgré la forme atypique des colonies des micro-organismes, une estimation précise de la surface des colonies est réalisée. Le suivi de la croissance de celles-ci a été effectué sur une durée de 65 heures. Les résultats obtenus sont présentés sous forme de courbes, objets des figures ci-jointes :

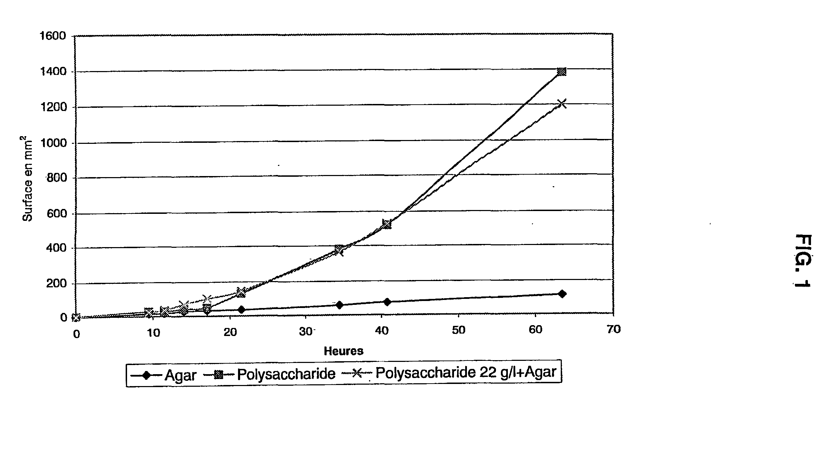

- la figure 1 représente les courbes de croissance de *Pseudomonas aeruginosa* sur chacun des trois milieux de croissance ci-dessus ;

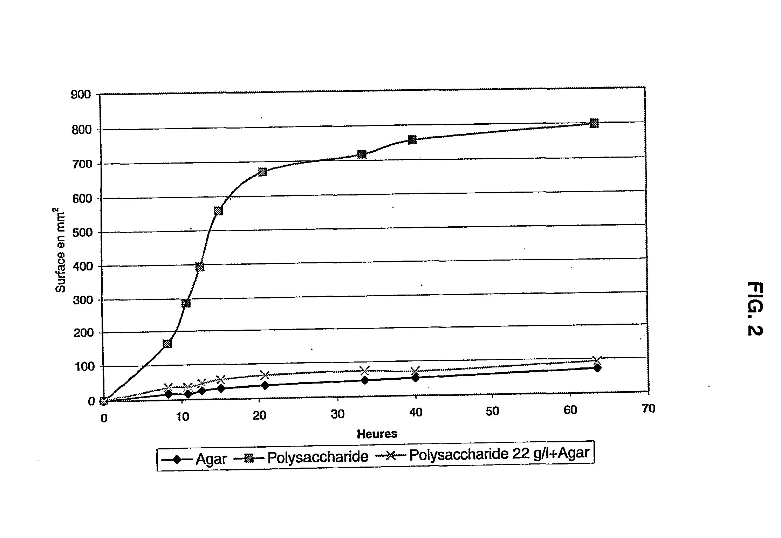

- la figure 2 représente les courbes de croissance de *Bacilis subtills* sur chacun des trois milieux de croissance ci-dessus ; et,

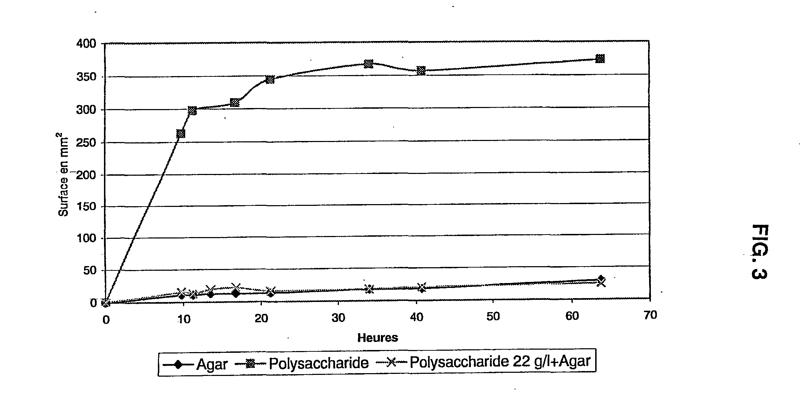

- la figure 3 représente les courbes de croissance de *Staphylococcus epidermidis* sur chacun des trois milieux de croissance ci-dessus.

Le milieu de culture selon la présente invention permet donc d'augmenter de façon très significative la taille des colonies de micro-organismes. Ainsi, de par cette augmentation de taille, il est possible d'identifier précocement les micro-organismes présents sur les boîtes.

On a également réalisé des essais comparatifs avec les mêmes micro-organismes que ci-dessus, mais avec des proportions différentes de polysaccharide, en ajoutant ou non 1g/l d'Agar-Agar ou 0,5 M de chlorure de sodium (NaCl). On a regroupé les résultats dans les tableaux 2 et 3 ci-après.

**Tableau 2**

| Surface des colonies en mm² après 63,5 heures de culture | | | |
|---|---|---|---|
| | *Bacilus subtilis* | *Pseudomonas aeruginosa* | *Staphylococcus epidermidis* |
| Agar | 72 | 115 | 29 |
| Polysaccharide | 798 | 1381 | 374 |
| Polysaccharide 22 g/l + NaCl | 755 | 1326 | 46 |
| Polysaccharide 22 g/l + Agar | 93 | 1201 | 25 |
| Polysaccharide 30 g/l | 206 | 2121 | 239 |
| Polysaccharide 30 g/l + Agar | 109 | 1379 | 38 |
| Polysaccharide 40 g/l | 179 | 2111 | 179 |
| Polysaccharide 40 g/l + Agar | 67 | 2197 | 34 |

**Tableau 3**

| Ratio surface témoin/surface essai | | | |
|---|---|---|---|
| | *Bacilus subtilis* | *Pseudomonas aeruginosa* | *Staphylococcus epidermidis* |
| Agar | 1 | 1 | 1 |
| Polysaccharide | 11 | 12 | 13 |
| Polysaccharide 22 g/l + NaCl | 11 | 12 | 2 |
| Polysaccharide 22 g/l + Agar | 1 | 10 | 1 |
| Polysaccharide 30 g/l | 3 | 18 | 8 |
| Polysaccharide 30 g/l + Agar | 2 | 12 | 1 |
| Polysaccharide 40 g/l | 2 | 18 | 6 |
| Polysaccharide 40 g/l + Agar | 1 | 19 | 1 |

Il apparaît de ces différents essais que la concentration croissante en polysaccharide ou l'ajout d'Agar-Agar à 1g/l tendent à atténuer l'écart entre le témoin et le milieu comprenant le polysaccharide à 22g/l. Il apparaît néanmoins que l'utilisation du polysaccharide favorise une rapide colonisation de la surface du milieu de culture quelle que soit sa concentration par rapport au milieu témoin.

Il n'échappera pas à l'homme du métier que, avantageusement, ce nouveau milieu de culture, de par l'utilisation du polysaccharide décrit dans le document FR-2 759 377-A, favorise une biodisponibilité optimale des substrats en facilitant la diffusion des matières dans la matrice du milieu de culture. Les micro-organismes en culture sur ce gel accèdent ainsi facilement aux substrats carboné et azoté, et aux facteurs de croissance : de ce fait, ils poursuivent leur croissance, alors que le milieu ne contenant que de l'Agar-Agar stoppe la croissance par épuisement des nutriments à la périphérie de la colonie.

L'accessibilité au substrat et à l'eau, qui résulte de l'aptitude du polysaccharide décrit dans le document FR-2 759 377-A à capter les liquides, permet de diminuer de façon importante l'affinité entre les cellules de micro-organismes et la surface du milieu gélosé. Cet abaissement de la tension entre les micro-organismes permet un étalement plus aisé des colonies et, par suite, une colonisation plus rapide de la surface, d'où la création de bio-films.

L'augmentation de la vitesse de croissance des micro-organismes permet ainsi la détection et l'identification précoce de micro-organismes pathogènes ou non lors d'analyses médicales et donc de définir un traitement ciblé plus précoce.

De plus, il est possible d'obtenir des colonies de micro-organismes génétiquement modifiés de tailles nettement supérieures à celles obtenues sur les milieux de culture habituellement utilisés. En effet, sur ces derniers, du fait de la fragilité et du petit nombre de telles souches et de l'épuisement rapide des nutriments à la périphérie des micro-organismes, les colonies obtenues sont difficilement détectables.

De même, le milieu de culture, objet de la présente invention, est particulièrement adapté à la conservation de collections de micro-organismes. En effet, sur des milieux de culture classiques, les micro-organismes se mettent rapidement sous forme résistante du fait de l'épuisement des nutriments facilement disponibles. Il est donc nécessaire de faire des repiquages fréquents des colonies en culture pour conserver les souches sous forme végétative.

Avec le milieu de culture selon la présente invention, l'augmentation de la disponibilité des nutriments fait que les micro-organismes cultivés sur ce gel restent sous forme végétative beaucoup plus longtemps, permettant d'espacer considérablement les repiquages de colonies.

De plus, le polysaccharide utilisé pour fabriquer le milieu de culture a une capacité de rétention de liquide considérable, ce qui permet d'éviter la formation de condensation sur le couvercle de la boîte de Pétri au cours de l'incubation. Ainsi, l'observation des colonies en culture en est facilitée et ceci d'autant plus que le milieu contenant le polymère est transparent. En outre, les risques de contamination sont diminués, puisqu'il n'est plus nécessaire d'ouvrir la boîte et de retirer la condensation avant observation.

Il va sans dire que l'utilisation de ce milieu est particulièrement adaptée pour les tests d'identification des modes respiratoires des micro-organismes, c'est-à-dire :
- aérobie stricte ;
- aérobie, anaérobie facultatif ; ou,
- anaérobie stricte.

Par exemple, l'inoculation de Saccharomyces cerevisae (micro-organisme aérobie anaérobie facultatif) et de *Botrytis* (micro-organisme aérobie stricte) sur un milieu à base viande/foie contenant le polysaccharide décrit dans le document FR-2 759 377-A, permet de vérifier les caractères respiratoires de ces micro-organismes avec un délai d'incubation plus court que sur un milieu de culture habituel.

Par ailleurs, certains micro-organismes, sur milieu de culture contenant le polysaccharide décrit dans le document FR-2 759 377-A, forment des colonies de formes et/ou couleurs caractéristiques. Ainsi, les tests d'identification sont facilités par une observation macroscopique préalable. La taxonomie de l'espèce étudiée est par conséquent plus aisée.

Enfin, la nature polysaccharidique de la molécule décrite dans le document FR-2 759 377-A et notamment la présence d'une molécule de pyruvate, d'acétate, de glucose et de galactose dans sa structure, fait de ce polysaccharide contenu dans le milieu de culture objet de la présente invention un substrat carboné utilisable par des micro-organismes possédant les enzymes d'hydrolyse de ce substrat.

## Revendications

1. Milieu de culture solide, **caractérisé par le fait qu'**il comprend, comme base gélifiante 2,2 à 6 % en poids d'un polysaccharide par rapport au poids total dudit milieu, lequel polysaccharide a une unité de reproduction qui possède une chaîne latérale et est constitué par six sucres neutres, dont le glucose et le galactose, et un sucre acide, des substituants pyruvates et acétates étant présents, et lequel polysaccharide forme un gel élastique et transparent lorsqu' il se trouve en solution dans l'eau, ou en solution saline à une concentration supérieure ou égale à 2 g/l.

2. Milieu de culture selon la revendication 1, **caractérisé par le fait que** le polysaccharide est synthétisé par un Rhizobium déposé sous le N°I-1809 auprès de la CNCM.

3. Milieu de culture selon l'une quelconque des revendications 1 à 2, **caractérisé par le fait qu'**il comprend aussi 0,5 M de chlorure de sodium.

4. Procédé d'obtention d'un milieu de culture solide pour les micro-organismes et les cellules eucaryotes selon l'une quelconque des revendications 1 à 3, **caractérisé en ce qu'**il comprend les étapes suivantes :
a) On mélange des milieux sous forme de poudre ;
b) On ajoute à ce mélange de 2,2 à 6% en poids d'un polysaccharide ayant une unité de reproduction qui possède une chaîne latérale et est constitué par six sucres neutres, dont le glucose et le galactose, et un sucre acide, des substituants pyruvates et acétates étant présents, une solution dans l'eau ou une solution saline à une concentration en ce polysaccharide supérieure ou égale à 2 g/l formant un gel élastique et transparent.
c) On met en solution le mélange ainsi obtenu ;
d) On stérilise à 110°C pendant 10 minutes ; et,
e) On coule dans des récipients ad hoc le produit stérilisé encore chaud.

5. Procédé selon la revendication 4, **caractérisé par le fait que**, à l'issue de l'étape e), on laisse reposer pendant 24h à température ambiante.

6. Procédé selon la revendication 4, **caractérisé par le fait que** l'on ajoute à la solution issue de l'étape c) 0,5 M de chlorure de sodium (NaCl).

## Patentansprüche

1. Festes Kulturmedium, **dadurch gekennzeichnet, dass** es als gelbildende Grundlage 2,2 bis 6 Gew.-% eines Polysaccharids, bezogen auf das Gesamtgewicht des Mediums, enthält, wobei das Polysaccharid eine Struktureinheit aufweist, die eine Seitenkette besitzt und aus 6 neutralen Zuckern, darunter Glucose und Galactose, und einem sauren Zucker zusammengesetzt ist, wobei Pyruvat- und Acetat-Substituenten vorliegen und wobei das Polysaccharid ein elastisches und durchsichtiges Gel bildet, wenn es sich in Lösung in Wasser oder in einer Salzlösung mit einer Konzentration von 2 g/Liter oder mehr befindet.

2. Kulturmedium nach Anspruch 1, **dadurch gekennzeichnet, dass** das Polysaccharid von einem Rhizobium synthetisiert wird, das bei CNCM unter der Nummer 1-1809 hinterlegt ist.

3. Kulturmedium nach einem der Ansprüche 1 und 2, **dadurch gekennzeichnet, dass** es ferner 0,5 M Natriumchlorid umfasst.

4. Verfahren zum Erhalten eines festen Kulturmediums für Mikroorganismen und eukaryontische Zellen nach einem der Ansprüche 1 bis 3, **dadurch gekennzeichnet, dass** es die folgenden Stufen umfasst:
a) man vermischt die Medien in Form von Pulver;
b) man versetzt das Gemisch mit 2,2 bis 6 Gew.-% eines Polysaccharids, das eine Struktureinheit aufweist, die eine Seitenkette besitzt und aus 6 neutralen Zuckern, darunter Glucose und Galactose, und einem sauren Zucker zusammengesetzt ist, wobei Pyruvat- und Acetat-Substituenten vorliegen und wobei das Polysaccharid ein elastisches und durchsichtiges Gel bildet, wenn es sich in Lösung in Wasser oder in einer Salzlösung mit einer Konzentration von 2 g/Liter oder mehr befindet.
c) man bringt das auf diese Weise erhaltene Gemisch in Lösung;
d) man sterilisiert 10 Minuten bei 110°C; und
e) man gießt das sterilisierte Produkt in noch heißem Zustand ad hoc in Behälter.

5. Verfahren nach Anspruch 4, **dadurch gekennzeichnet, dass** man das Produkt am Ende von Stufe e) 24 Stunden bei Umgebungstemperatur ruhen lässt.

6. Verfahren nach Anspruch 4, **dadurch gekennzeichnet, dass** man die Lösung am Ende der Stufe c) mit 0,5 M Natriumchlorid (NaCl) versetzt.

## Claims

1. A solid culture medium, **characterised by** the fact that it comprises, as a gelling base, 2.2 to 6 % by weight of a polysaccharide with respect to the total weight of said medium, which polysaccharide has a reproduction unit which has a side chain and comprises six neutral sugars, including glucose and galactose, and an acid sugar, pyruvate and acetate substituents being present, and which polysaccharide forms an elastic, transparent gel when in solution in water or in a saline solution having a concentration greater than or equal to 2 g/l

2. A culture medium according to claim 1, **characterised by** the fact that the polysaccharide is synthesised by a Rhizobium registered under the No. 1-1809 with the CNCM.

3. A culture medium according to either of claims 1 or 2, **characterised by** the fact that it also comprises 0.5 M sodium chloride.

4. A method for obtaining a solid culture medium for micro-organisms and eukaryotic cells according to any one of claims 1 to 3, **characterised in that** it comprises the following steps:
a) media are mixed in powder form;
b) added to this mixture are 2.2 to 6 % by weight of a polysaccharide having a reproduction unit which has a side chain and comprises six neutral sugars, including glucose and galactose, and an acid sugar, pyruvate and acetate substituents being present, a solution in water or a saline solution having a concentration of this polysaccharide which is greater than or equal to 2 g/l forming an elastic, transparent gel.
c) the mixture thus obtained is dissolved;
d) sterilisation takes place for 10 minutes at 110°C; and,
e) the still hot sterilised product is poured as appropriate into containers.

5. A method according to claim 4, **characterised by** the fact that at the end of stage e), it is allowed to rest for 24 hours at ambient temperature.

6. A method according to claim 4, **characterised by** the fact that 0.5 M sodium chloride (NaCl) is added to the solution from stage c).
